# EUROPÄISCHE PATENTSCHRIFT

(11) **EP 2 004 131 B1**
(45) Veröffentlichungstag und Bekanntmachung des Hinweises auf die Patenterteilung: **05.10.2011**
(21) Anmeldenummer: 07727365.4
(22) Anmeldetag: 27.03.2007
(51) Int. Cl.: A61K 6/093

(54) **DENTALMATERIALIEN ENTHALTEND HYDROPHOBE, NANOPARTIKULÄRE KIESELSÄURECOKONDENSATE UND DEREN VERWENDUNG**
DENTAL MATERIALS CONTAINING HYDROPHOBIC NANOPARTICULATE SILICIC ACID CO-CONDENSATES AND USE THEREOF
MATÉRIAUX DENTAIRES CONTENANT DES COCONDENSATS D'ACIDE SILICIQUE HYDROPHOBES ET NANOPARTICULAIRES ET UTILISATION DESDITS MATÉRIAUX

(30) Priorität: 07.04.2006 DE 102006016474
(43) Veröffentlichungstag der Anmeldung: 24.12.2008
(73) Patentinhaber: Ivoclar Vivadent AG, 9494 Schaan (LI)
(72) Erfinder: MOSZNER, Norbert, 9495 Triesen (LI); RHEINBERGER, Volker, 9490 Vaduz (LI); KLAPDOHR, Simone, 83022 Rosenheim (AT); FISCHER, Urs-Karl, 9320 Arbon (CH)
(74) Vertreter: Fitzner, Uwe
(86) Internationale Anmeldenummer: PCT/EP2007/052892
(87) Internationale Veröffentlichungsnummer: WO 2007/115926

(56) Entgegenhaltungen:
- EP-A- 0 451 709
- EP-A- 0 804 919
- DE-A1- 19 903 177
- DE-A1- 19 910 895

## Beschreibung

Die vorliegende Erfindung betrifft Dentalmaterialien enthaltend Kieselsäureesterkondensate sowie deren Verwendung.

Die Hydrolyse und Kondensation von Tetraalkoxysilanen führt bekanntlich (vgl. M.A. Brook, Silicon in Organic, Organometallic, and Polymer Chemistry, John Wiley& Sons, New York etc. 2000, 312-314) je nach den gewählten Bedingungen, d.h. in Abhängigkeit vom pH-Wert, zu einer unterschiedlichen Morphologie der Produkte, die aus den sog. Q-Einheiten (SiO₂) aufgebaut sind. So bilden sich unter sauren Bedingungen schwach verzweigte Polymerstrukturen, die bei hohem Umsatz vernetzen können. Demgegenüber ist unter basischen Bedingungen die Hydrolyse zwar langsamer, aber die beschleunigte Kondensation führt schon initial zur Entstehung von dreidimensionalen Clustern und schließlich zu dichten SiO₂-Partikeln. Dementsprechend lassen sich nach Stöber, Fink und Bohn (vgl. J. Colloid Interface Sci. 26 (1968) 62-69) monodisperse kugelförmige SiO₂-Partikel im Bereich zwischen 10 und 500 nm, sog. SiO₂-Nanospheres, gezielt durch hydrolytische Kondensation von Kieselsäuretetraethylester (TEOS) in basischer Lösung (Ammoniak) von Wasser und Alkoholen herstellen. Dabei lässt sich die Partikelgröße durch die Auswahl der Reaktionsbedingungen kontrollieren. Eng verteilte kleine Partikel von z.B. ca. 5 bis 8 nm Durchmesser wurden vor allem in einer schnellen Reaktion von TEOS in Ethanol erhalten. Nach Petroso et al. (Colloid Polymer Science 282 (2003) 19-26) lassen sich durch Cokondensation von TEOS mit Methyltriethoxysilan (MTEOS) oder Dimethyldiethoxysilan (DMDEOS) und anschließender Umsetzung mit Trimethylchlorsilan in n-Hexan SiO₂-Nanospheres mit einem Durchmesser im Bereich von 10-24 nm herstellen, die nach ihrer Isolierung als Pulver vollständig in Hexan dispergiert werden können, obwohl sie 19-40 % des Siliciums als SiO₂-Strukturen enthalten.

Bei der Kondensation von organisch modifizierten Trialkoxysilanen bilden sich meist hochviskose Produkte, die als Silsequixoxane (RSiO_{3/2}, T-Strukturen) oder auch als Ormosile (organically modified Silicates) bezeichnet werden und aus mehr oder weniger regelmässig aufgebauten prismatischen Käfigstrukturen oder Leiterstrukturen bestehen (vgl. M.A. Brook, Silicon in Organic, Organometallic, and Polymer Chemistry, John Wiley& Sons, New York etc. 2000, 320-324). Typisch niedrigviskose Polysiloxane, wie z.B. Polydimethylsiloxan, werden ausgehend von difunktionellen Silanen hergestellt und sind meist aus linearen Polymeren mit sog. D-Strukturen, R₂SiO, aufgebaut (vg. J.E. Mark, H. R. Allcock, R. West, Inorganic Polymers, Prentice Hall, Englewood Cliffs 1992, 141-145).

Aus der DE 198 16 148 A 1 sind Organopolysiloxanpartikel enthaltende acrylathaltige Zusammensetzungen bekannt, wobei die Organopolysiloxanpartikel aus einem einzigen Molekül bestehen. Die Partikel weisen einen mittleren Durchmesser von 5 bis 200 nm auf und sind in einem Lösemittel (z.B. Toluol, Tetrahydrophoran oder Wasser) bei 20 °C zumindest 5 Gew.-% löslich. Die betreffenden Zusammensetzungen können in Dentalmaterialien Verwendung finden. Ziel der DE 198 16 148 A1 ist es, Organopolysiloxannanopartikel zur Verfügung zu stellen, die als Füllstoff, also als feste Komponente verwendbar sind.

Aus der DE 694 25 473 T3 ist ein Verfahren zur Herstellung von organfunktionellen Gruppen enthaltenden Organopolysiloxanen und daraus hergestellten Organopolysiloxanen, Mercapto- und Alkoxygruppen enthaltende Organopolysiloxane und Verfahren zu deren Herstellung bekannt. Dabei ist das Wesentliche, dass die Silanhydrolyse und die Polykondensation in Anwesenheit einer im wesentlichen neutralen Fluor enthaltenden Verbindung durchgeführt wird. Bei dieser Hydrolyse und Kondensation entstehen hochporöse Feststoffe.

Die DE 390 034 07 A1 beschreibt ein dentales Füllmaterial, das mindestens ein Polysiloxan oder Heteropolysiloxan mit polymerisierbaren (meth)Acrylestergruppen enthält, die durch Kondensation von Tetraalkyloxysilanen mit Tri- oder Dialkoxysilanen und/oder Titan- bzw. Zirkoniumtetraalkoxiden zugänglich sind. Es handelt sich bei den beschriebenen Materialien um als Füllstoffe genutzte Feststoffe. Das Vorliegen von Nanopartikeln oder flüssigen Verbindungen, die als Verdünner geeignet sind, ist nicht beschrieben.

Das Dokument EP 0 804 919 A offenbart die Verwendung einer Zusammensetzung als Dentalmaterial sowie ein Dentalmaterial und insbesondere die Verwendung solcher Zusammensetzungen, die ein Kieselsäurekondensat eines hydrolysierbaren und polymerisierbaren Norbornensilans oder ein Kieselsäurekondensat eines hydrolysierbaren und polymerisierbaren Mercaptosilans zusammen mit einem Reaktionspartner für eine Thiol-En-Polymerisation enthalten.

Der Erfindung liegt die Aufgabe zugrunde, Dentalmaterialien bereitzustellen, die im Vergleich zu rein auf Dimethacrylaten basierenden Dentalmaterialen annähernd gleiche radikalische Photopolymerisationsreaktivität und mechanische Eigenschaften zeigen, jedoch Verdünner mit geringerer Cytotoxizität, verminderte Löslichkeit in Wasser und einen Brechungsindex kleiner 1,45 enthalten.

Diese Aufgabe wird gelöst durch Dentalmaterialien enthaltend flüssige, nanopartikuläre, funktionalisierte hydrophobe Cokondensate von Kieselsäuretetraestern mit funktionalisierten Trialkoxysilanen der Formel I

**P-(CH₂)ₙ-Si(OR)₃**

wobei P, R und n unabhängig voneinander die folgenden Bedeutungen haben:
P = eine polymerisationsfähige CH₂=CH-, CH₂=CH-O- Styryl-, (Meth)acryl-, (Meth)acrylamid- oder eine polyadditionsfähige SH-Gruppe darstellt,
R = Methyl, Ethyl oder Propyl bedeutet und
n = von 3 bis 15 ist,
herstellbar durch Cokondensation von Kieselsäuretetraalkylestern mit in ω-Stellung, d.h. in Endstellung, funktionalisierten Alkyltrialkoxysilanen.

Bevorzugte Trialkoxysilane sind solche, bei denen mindestens eine der Variablen der Formel (I) die vorstehend beschriebene bevorzugte Definition aufweist.

Besonders bevorzugte erfindungsgemässe Silane sind solche bei denen:
- P: = eine polymerisationsfähige CH₂=CH-, (Meth)acryl-, (Meth)acrylamid- oder eine polyadditionsfähige SH-Gruppe darstellt,
- R: = Methyl oder Ethyl- bedeutet und
- n: = von 3 bis 10 ist.

Vorzugsweise sind die Kondensate, die mit Trimethylsilylgruppen endgruppenfunktionalisiert.

Als Silylierungsmittel kommen neben Trimethylchlorsilan andere bekannte Reagentien zur Einführung der Trimethylsilylgruppe in Betracht, wie z.B. Trimethylmethoxysilan, Trimethylacetoxysilan oder Hexamethyldisilazan. Erfindungsgemäss werden vorzugsweise als Kieselsäuretetraalkylester Kieselsäuretetramethyl- oder -ethylester eingesetzt.

Die Kondensation wird vorzugsweise unter basischen Bedingungen durchgeführt. Besonders bevorzugt sind Bereiche des pH-Wertes von 7.5 bis 13 ganz besonders bevorzugt von 7.5 bis 11.

Die hydrophoben Kieselsäureesterkondensate sind in aliphatischen Alkanen löslich. Insbesondere kommen Alkane von C₅ bis C₁₂. in Betracht. Besonders bevorzugt sind die Kondensate in Hexan, Heptan, Octan oder Nonan löslich. Ganz besonders bevorzugt sind in Hexan lösliche Kondensate.

Die erfindungsgemäßen flüssigen, in Hexan löslichen, nanopartikulären, funktionalisierten hydrophoben Kieselsäurecokondensate lassen sich so herstellen, dass im ersten Schritt eine hydrolytische Kondensation von TEOS oder Kieselsäuretetramethylester (TMOS) bzw. dessen Mischungen mit den Trialkoxysilanen der Formel I bzw. Mischungen davon in Alkohol, vorzugsweise in Ethanol oder Methanol bei 30-80 °C, vorzugsweise bei 40 bis 80°C, ganz besonders bevorzugt bei 50 bis 60 °C unter Verwendung eines Katalysators, vorzugsweise einer wässrigen NH₄OH-Lösun durchgeführt wird.

Erfindungsgemäß wird eine Wassermenge von 0.5 bis 3.0 mol pro Alkoxysilangruppe (SiOR) zugesetzt. Besonders bevorzugt sind Wassermengen von 2.0 bis 3.0 mol pro Alkoxysilangruppe.

Das Verhältnis von Kieselsäuretetraalkylester zu Trialkoxysilanen der Formel I kann variiert werden. Vorzugsweise kann es im Bereich von 0,5 bis 1,2 variiert werden. Besonders bevorzugt sind Bereiche von 0.5 bis 1.0.

Im zweiten Schritt wird dann die alkoholische Lösung des hydrolytischen Kondensates mit einer Lösung eines Silylierungsmittels zur Einführung von Trimethylsilylgruppen, vorzugsweise von Trimethylchlorsilan (TMCS) in einem aliphatischen Alkan, vorzugsweise Alkanen von C₅ bis C₁₂, besonders bevorzugt Hexan, Heptan oder Nonan über einen Zeitraum von 10 bis 40 h, vorzugsweise von 10 bis 30 h, ganz besonders bevorzugt von 10 bis 20 umgesetzt, wobei die TMCS-Menge im Bereich von 10 bis 40 mol-%, vorzugsweise 20 bis 40 mol-%, ganz besonders bevorzugt 25 bis 35 mol-% der im ersten Schritt hydrolysierten Si-OR-Menge variieren kann. Die Isolierung der flüssigen, nanopartikulären, vorzugsweise in Alkan löslichen, funktionalisierten hydrophoben Kieselsäurecokondensate kann durch Abtrennung der Alkoholphase von der Alkanphase, vorzugsweise Hexanphase zusätzliche Extraktion der Alkoholphase mit Alkan, vorzugsweise Hexan, Abdestillieren des Lösungsmittels von den vereinigten Hexanphasen und Trocknung des Rückstandes im Hochvakuum erfolgen. Dabei hat die ²⁹Si-NMR-spektroskopische Untersuchung der gebildeten flüssigen Kondensate das Vorhandensein eines erheblichen Anteils von hochkondensierten Q-Strukturen (SiO₂-Einheiten) ergeben, was eine nanopartikuläre Struktur der in Alkan löslichen, funktionalisierten hydrophoben Kieselsäurecokondensate belegt. Die Größe der Partikel variierte im Bereich von 1 bis 5 nm, wobei die Partikelgrößenbestimmung durch dynamische Lichtstreuung erfolgte.

Die erfindungsgemässen Dentalmaterialien auf der Basis der flüssigen, nanopartikulären, vorzugsweise in Alkan löslichen, funktionalisierten hydrophoben Kieselsäurecokondensate lassen sich vorzugsweise mit den bekannten radikalischen Initiatoren (vgl. Encyclopedia of Polymer Science and Engineering, Vol. 13, Wiley-Intersci. Pub., New York etc. 1988, 754ff.) polymerisieren. Es eignen sich besonders Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acyl- der Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon.

Weiterhin können auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, tert.-Butylperoctoat, tert.-Butylperbenzoat oder Di-(tert.-butyl)-peroxid verwendet werden. Als Initiatoren für die Heißhärtung eignen sich auch Benzpinakol und 2,2'-Dialkylbenzpinakole.

Zur Beschleunigung der Initiierung von Peroxiden oder α-Diketonen können auch häufig Kombinationen mit aromatischen Aminen bevorzugt werden. Redoxsysteme, die sich bereits bewährt haben, sind: Kombinationen aus Benzoylperoxid oder Campherchinon mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und solchen Reduktionsmitteln, wie z.B. Ascorbinsäure, Barbiturate oder Sulfinsäuren, geeignet. Die erfindungsgemässen Dentalmaterialien können in Mischung mit herkömmlichen radikalisch polymerisierbaren Monomeren, insbesondere mit difunktionellen (Meth)-acrylat-Vernetzern eingesetzt werden. Diesbezüglich eignen sich vor allem vernetzende bi- oder mehrfunktionelle Acrylate bzw. Methacrylate wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi-(meth)acrylat, Trimethylolpropantri(meth)acrylat, Pentaerythrittetra(meth)acrylat, sowie Butandioldi(meth)acrylat, 1,10-Decandioldi(meth)acrylat, 1,12-Dodecandioldi(meth)acrylat. Weiterhin lassen sich auch als Comonomere besonders methacrylatmodifizierte Polysiloxan-Harze verwenden, die durch hydrolytische Kondensation von entsprechenden methacrylatgruppenhaltigen Silanen zugänglich sind. Diese methacrylatmodifizierten Polysiloxan-Harze zeichnen sich durch eine hohe Funktionalität aus, da sie meist, Cokondensate mit nichtfunktionalisierten Silanen ausgenommen, pro Siloxan-Wiederholungseinheit mindestens eine polymerisationsfähige Gruppe tragen. Geht man von Silanen mit zwei Methacrylatgruppen aus, so enthält jede Siloxaneinheit zwei polymerisationsfähige Gruppen. Die Polysiloxane sind meist hochviskos und können dementsprechend mit den erfindungsgemässen flüssigen, in Hexan löslichen, funktionalisierten hydrophoben Kieselsäurecokondensaten wirkungsvoll verdünnt werden. Analog zu den erfindungsgemässen flüssigen, nanopartikulären, vorzugsweise in Alkan löslichen, funktionalisierten hydrophoben Kieselsäurecokondensaten zeichnen sich Polysiloxane auch durch eine geringe Löslichkeit in Wasser oder wässrigen Lösungen aus, so dass auch geringste nichtpolymerisierte Anteile aus dem Dentalmaterial unter oralen Bedingungen nicht löslich und damit auch nicht auswaschbar sind. Für die Herstellung der polymerisierbaren Polysiloxan-Harze geeignete Methacrylsilane sind kommerziell zugänglich, wie z.B. 3-(Methacryloyloxy)propyltrimethoxysilan (MEMO), oder lassen sich z.B. durch Umsetzung von Glycerindimethacrylat mit 3-Isocyanatopropyltriethoxysilan (DMAURS), EP 0 618 242 B1) oder von Glycerindimethacrylat mit Glutarsäureanhydrid und anschließend mit 3-Aminopropyltriethoxysilan (DMAGAMS,

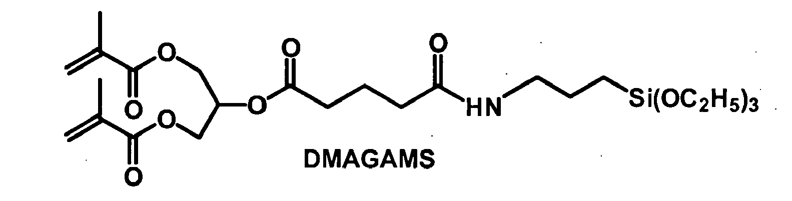

DE 199 03 177 C2) einfach herstellen, wobei in den angegebenen Patenten auch die Herstellung der methacrylatmodifizierten Polysiloxan-Harze durch hydrolytische Kondensation der methacrylatgruppenhaltigen Silane beschrieben ist.

Als Reaktionskomponente für Polyadditionen mit flüssigen, nanopartikulären, vorzugsweise in Alkan löslichen, SH-funktionalisierten hydrophoben Kieselsäurecokondensate lassen sich di- oder multifunktionellen Acrylate einsetzen. Beispiele für geeignete Acrylate sind Ethylenglycoldiacrylat, Hexandioldiacrylat, Tripropylenglycoldiacrylat, ethoxyliertes Bisphenol-A-Diacrylat, Polyethylenglycol-200-diacrylat, Trimethylolpropantriacrylat, Pentaerythrittetraacrylat. Als geeignete Katalysatoren für die Polyaddition werden vorzugsweise Alkalimetallhydroxide, wie z.B. KOH, Tetraalkylammoniumhydroxide, z.B. Tetrabutylammoniumhydroxid, insbesondere bicyclische Amidine, wie 1,5-Diazabicyclo[4.3.0]-5-nonen oder 1,8-Diazabicydo[5.4.0]-7-undecen, und Guanidine, vor allem Tetramethylguanidin eingesetzt.

Weiterhin können die erfindungsgemässen Dentalmaterialien auf der Basis der flüssigen, nanopartikulären, vorzugsweise in Alkan löslichen, funktionalisierten hydrophoben Kieselsäurecokondensate zur Verbesserung der mechanischen Eigenschaften mit organischen oder anorganischen Partikeln gefüllt werden. Bevorzugte anorganische Füllstoffe sind amorphe kugelförmige nanopartikuläre Füllstoffe auf der Basis von Oxiden, wie pyrogene Kieselsäure oder Fällungskieselsäure, ZrO₂ und TiO₂ bzw. Mischoxiden aus SiO₂, ZrO₂ und/oder TiO₂ mit einem mittleren Partikeldurchmesser von 10 bis 200 nm, mikrofeine Füllstoffe sowie Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch Glasfasern, Polyamid- oder Kohlenstoff-Fasern eingesetzt werden.

Schließlich lassen sich den erfindungsgemässen Dentalmaterialien auf der Basis der flüssigen, nanopartikulären, vorzugsweise in Alkan löslichen, funktionalisierten hydrophoben Kieselsäurecokondensate im Bedarfsfalle weitere Komponenten zusetzen, wie z.B. Stabilisatoren, UV-Absorber, Farbstoffe oder Pigmente sowie Gleitmittel.

Auf der Basis der erfindungsgemässen flüssigen, nanopartikulären, in Hexan löslichen, funktionalisierten hydrophoben Kieselsäurecokondensate lassen sich besonders Dentalmaterialien, wie Füllungskomposite, Befestigungszemente oder Materialien für Beschichtungen herstellen. D.h., die erfindungsgemäßen Dentalmaterialien können für Füllungskomposite, Befestigungszemente oder Materialien für Beschichtungen verwendet werden. Solche Materialien zeichnen sich durch eine geringe Wasseraufnahme, gute mechanische Eigenschaften, hohe Abrasionsfestigkeit und Biokompatibilität aus.

Vorzugsweise werden die erfindungsgemäßen Trialkoxysilane für Füllungskomposite eingesetzt, enthaltend
a) 1 bis 45 Gew.-%, und besonders bevorzugt 1,00 bis 30 Gew.-% an in Hexan löslichen, nanopartikulären, funktionalisierten, hydrophoben Kieselsäurecokondensaten,
b) 0,01 bis 5 Gew.-%, besonders bevorzugt 0,1 bis 2,0 Gew.-% an radikalischem Initiator,
c) 5 bis 50 Gew.-% und besonders bevorzugt 0 bis 40 Gew.-% an anderen radikalisch polymerisierbaren Monomerkomponenten,
d) 30 bis 85 Gew.-% und besonders bevorzugt 40 bis 80 Gew.-% an einem Füllstoff.

Weiterhin können die erfindungsgemäßen Dentalmaterialien für Zemente verwendet werden, enthaltend:
a) 1 bis 60 Gew.-%, und besonders bevorzugt 1,0 bis 30 Gew.-% an in Hexan löslichen, nanopartikulären, funktionalisierten, hydrophoben Kieselsäurecokondensaten,
b) 0,01 bis 5 Gew.-%, besonders bevorzugt 0,1 bis 2,0 Gew.-% an radikalischem Initiator,
c) 5 bis 60 Gew.-% und besonders bevorzugt 0 bis 40 Gew.-% an anderen radikalisch polymerisierbaren Monomerkomponenten,
d) 20 bis 60 Gew.-% und besonders bevorzugt 30 bis 60 Gew.-% an einem Füllstoff.

Eine weitere vorteilhafte Verwendung der erfindungsgemäßen Dentalmaterialien sind Beschichtungsmaterialien enthaltend:
a) 1 bis 95 Gew.-%, und besonders bevorzugt 10 bis 40 Gew.-% an in Hexan löslichen, nanopartikulären, funktionalisierten, hydrophoben Kieselsäurecokondensate,
b) 0,01 bis 5 Gew.-%, besonders bevorzugt 0,1 bis 2,0 Gew.-% an radikalischem Initiator,
c) 5 bis 60 Gew.-% und besonders bevorzugt 0 bis 40 Gew.-% an anderen radikalisch polymerisierbaren Monomerkomponenten,
d) 0 bis 20 Gew.-% an einem Füllstoff.

Die Erfindung wird im Folgenden anhand von Beispielen näher erläutert.

### Beispiel 1: Synthese des in Hexan löslichen, mit Methacrylatgruppen funktionalisierten hydrophoben Kieselsäurecokondensates McNano-1

0.06 mol Tetraethoxysilan (TEOS) und 0.06 mol 3-Methacryloxypropyltriethoxysilan werden mit 150 ml Methanol vermischt und auf 50 °C erhitzt. Zum Gemisch werden 1.116 mol (r = 9.3 mol H₂O/m Si) einer 0.1 N NH₄OH-Lösun zugegeben und 3 h bei 50°C gerührt. 0.134 mol Trimethylchlorsilan (TMCS) in 200 ml n-Hexan werden anschließend bei Raumtemperatur langsam zugegeben und 18 h gerührt. Die Methanolphase wird abgetrennt und mit n-Hexan extrahiert, die vereinigten n-Hexan-Phasen werden mit Wasser gewaschen und über wasserfreiem Natriumsulfat getrocknet. Nach Entfernung des Lösungsmittels am Rotationsverdampfer und Trocknung bei 40 °C (10-¹mbar) werden 11.8 g (49.8% d. Th.) einer farblosen, klaren Flüssigkeit erhalten.

Charakterisierung: ¹H-NMR-Spektroskopie: Verhältnis Methacrylatgruppen zu Trimethylsilylgruppen = 1:1.96; ²⁹Si-NMR-Spektrum: Verhältnis R₃SiO_{0.5}: RSiO₁._{5:} SiO₂ = 1:0.56:0.34; keine Monomeren enthalten; Scherviskosität: 0.10 Pa·s; Glührückstand: 36.9 Gew.-%; Elementaranalyse: C-Gehalt: 39.8 Gew.-%; Brechungsindex: 1.4346, Partikelgrößenbestimmung durch dynamische Lichtstreuung mittels des Malvern Zeta Sizer Nano einer 1,0 %-igen Ethanol Lösung des Kondensates McNano-1 ergab eine mittlere Partikelgröße von 1,5 nm.

Das Beispiel zeigt, dass trotz der Verwendung der Mischung des tetrafunktionellen TEOS und des trifunktionellen 3-Methacryloxypropyltriethoxysilans sowie basischer Kondensationsbedingungen sich ein flüssiges, sehr niedrigviskoses, nanopartikuläres Kieselsäurecokondensat gebildet hat.

### Beispiel 2: Synthese des Silans Si-87 (1,3-Dimethacryloyloxypropyl-[4-(3-triethoxysilyl)propyl-N-methylaminocarbonyl)]butyrat) und dessen hydrolytische Kondensation zum Harz OM-87

17,12 g (50 mmol) 1,3-Dimethacryloyloxypropyl-2-yl-hydrogenglutarat (= 1:1-Umsetzungsproduktes von Glycerindimethacrylat mit Glutarsäureanhydrid) werden in 65 ml trocknem Methylenchlorid gelöst, mit 0,2 g 4-Dimethylaminopyridin versetzt und auf 0°C abgekühlt. Dazu gibt man portionsweise 9,59 g (50 mmol) N-(3-Dimethylaminopropyl-N'-ethylcarbodiimidhydrochlorid, so dass die Temperatur nicht 0 °C übersteigt. Nach Erwärmen auf Raumtemperatur und 1 h Rühren, wird das Reaktionsgemisch auf Eis geschüttet und der pH-Wert der eiskalten Mischung auf 6-7 mit 0,3 N Salzsäure eingestellt. Anschließend wird die Methylenchloridphase 3-mal mit 150 ml Eiswasser gewaschen und über wasserfreien Natriumsulfat getrocknet. Nach Abdestillieren des Lösungsmittels im Vakuum werden 21,9 g (80.8% d. Th.) des Silans Si-87 als leicht gelbliche, klare Flüssigkeit erhalten.

Zur hydrolytischen Kondensation werden 10,0 g Si-87 in 90 g trockenem Ethylacetat gelöst. Hierzu werden 1, 04 g einer wässrigen 1N NH₄F-Lösung zugegeben und die Lösung wird 48 h bei Raumtemperatur gerührt. Nach Filtration über eine Glasfritte stabilisiert man die Lösung mit 0.5 mg 2,6-Di-tert.-butyl-4-(dimethylaminomethyl)phenol und 1mg 2,6-Di-tert.-butyl-4-methylphenol. Die leicht gelblich trübe Lösung wird bei 40°C am Rotationsverdampfer unter Lufteinleitung vom Lösungsmittel befreit und anschließend im Feinvakuum bei 40°C und 0.06 mbar getrocknet. Man erhält 8,64 g (93.5% d. Th.) des hochviskosen (η= 407 Pa·s) Harzes OM-87.

### Beispiel 3: Synthese eines in Hexan löslichen, mit Mercaptogruppen funktionalisierten hydrophoben Kieselsäurecokondensates

0.06 mol Tetraethoxysilan und 0.06 mol 3-Mercaptopropyltriethoxysilan werden mit 150 ml Methanol vermischt und auf 50 °C erhitzt. Zum Gemisch werden 1.116 mol (r = 9.3 mol H₂O/m Si) einer 0.1N NH₄OH-Lösung zugegeben und 3 h bei 50°C gerührt. 0.134 mol TMCS in 200 ml n-Hexan werden anschließend bei Raumtemperatur langsam zugegeben und 18 h gerührt. Die Methanolphase wird abgetrennt und mit n-Hexan extrahiert, die vereinigten n-Hexan-Phasen werden mit Wasser gewaschen und über wasserfreiem Natriumsulfat getrocknet. Nach Entfernung des Lösungsmittels am Rotationsverdampfer und Trocknung bei 40 °C (10⁻¹ mbar) wird eine farblose, klare Flüssigkeit (15.9 g (72.1 % d. Th.)) erhalten.

Charakterisierung: ¹H-NMR-Spektrum: Verhältnis Mercaptogruppen zu Trimethylsilylgruppen = 1: 2.11; ²⁹Si-NMR-Spektrum: Verhältnis R₃SiO_{0.5}: RSiO₁.₅: SiO₂ = 1:0.49:0.44; keine Monomeren enthalten; Scherviskosität: 0.12 Pa·s; Glührückstand: 38.6 Gew.-%; Brechungsindex: 1.4390.

### Beispiel 4: Synthese des in Hexan löslichen, mit Methacrylatgruppen funktionalisierten hydrophoben Kieselsäurecokondensates McNano-2 ausgehend von einem langkettigen Methacryloylsilan

0.03 mol TEOS und 0.03 mol 10-(Methacryloxy)-decyltriethoxysilan werden mit 75 ml Methanol vermischt und auf 50 °C erhitzt. Zum Gemisch werden 0.56 mol·(r = 9.3 mol H₂O/mol Si) einer 0.1 N NH₄OH-Lösun zugegeben und 3 h bei 50 °C gerührt. 67 mmol TMCS in 100 ml n-Hexan werden anschließend bei Raumtemperatur langsam zugegeben und 17 h gerührt. Das Gemisch wird kurz filtriert um einige grobe Feststoffteilchen abzutrennen. Anschließend wird die Methanolphase abgetrennt und mit n-Hexan extrahiert, die vereinigten n-Hexan-Phasen werden mit Wasser gewaschen und über wasserfreiem Natriumsulfat getrocknet. Nach Entfernung des Lösungsmittels am Rotationsverdampfer und Trocknung bei 40 °C (10⁻¹ mbar) werden 11.7 g (77% d. Th.) einer farblosen, klaren Flüssigkeit erhalten.

Charakterisierung: ¹H-NMR-Spektroskopie: Verhältnis Methacrylatgruppen zu Trimethylsilylgruppen = 1:2.4; ²⁹Si-NMR-Spektrum: Kondensationsgrad der T-Einheiten: 83%; keine Monomeren enthalten; Scherviskosität: 56 mPa·s; Brechungsindex: 1.4332, Partikelgrößenbestimmung durch dynamische Lichtstreuung mittels des Malvern Zeta Sizer Nano-S einer 1,0 %-igen Ethanol Lösung des Kondensates McNano-2 ergab eine mittlere Partikelgröße von 3,7 nm.

Das Beispiel zeigt, dass sich bei Verwendung eines langkettigen hydrohoben Silans die Ausbeute an flüssigen, sehr niedrigviskosen, nanopartiuären Kieselsäurecokondensat-Harz deutlich erhöht.

### Beispiel 5: Herstellung eines Komposites mit dem mit Methacrylatgruppen funktionalisierten hydrophoben Kieselsäurecokondensat McNano-1 aus dem Beispiel 1

Entsprechend der nachfolgend aufgeführten Tabelle 1 wurde ein Komposit auf der Basis von A eines reinen Methacryltrialkoxysilan-Polykondensates OM-87 (aus Beispiel 2) und B unter Einbeziehung des mit Methacrylatgruppen funktionalisierten hydrophoben Kieselsäurecokondensat es

**Tabelle1: Zementzusammensetzung**

| Stoffe | Material A Anteile (Gew.-%) | Material B Anteile (Gew.-%) |
|---|---|---|
| OM-87 | 29,8 | 23,9 |
| McNano-1 | - | 5,9 |
| Photoinitiator¹) | 0,2 | 0,2 |
| Sphärosil²) | 12,3 | 12,3 |
| Glasfüller 27884³⁾ | 44,1 | 44,1 |
| Aerosil OX-50⁴⁾ | 0,9 | 0,9 |
| Ytterbiumtrifluorid⁵⁾ | 12,7 | 12,7 |

| | | |
|---|---|---|
| ¹⁾ 1:1-Mischung aus Campherchinon und p-Dimethylaminobenzoesäureethylester ²⁾ SiO₂-ZrO₂-Mischoxid, 35 Gew.-% ZrO₂ Partikelgröße: 130-230 nm (Tokoyama Soda, Japan) ³⁾ Silanisierter Bariumaluminiumborosilikat Glasfüller mit einer mittleren Partikelgröße von 1,2 µm (Schott, Landshut) ⁴⁾ Silanisierte pyrogene Kieselsäure mit einer Primärpartikelgröße von 40 nm (Degussa) ⁵⁾ YbF3 mit einer Partikelgröße von 0,3-0,5 µm (Rhodia) | | |

**Tabelle 2: Kompositeigenschaften**

| Materialeigenschaft | Material A | Material B |
|---|---|---|
| Biegefestigkeit (MPa) nach 24 h | 62 | 74 |
| Biegefestigkeit (MPa) nach 24 h WL¹⁾ | 51 | 75 |
| Biegefestigkeit (MPa) nach 7 d WL | 60 | 76 |
| Biege-E-Modul (GPa) nach 24 h | 5,84 | 5,25 |
| Biege-E-Modul (GPa) nach 24 h WL | 5,41 | 5,24 |
| Biege-E-Modul (GPa) nach 7 d WL | 6,16 | 5,95 |

| | | |
|---|---|---|
| ¹⁾ WL = Wasserlagerung der Prüfkörper bei 37°C | | |

McNano-1 aus Beispiel 1 mittels eines Walzenstuhles "Exakt" (Exakt Apparatebau, Norderstedt) hergestellt. Von den Materialien wurden entsprechende Prüfkörper präpariert, die 2 mal 3 Minuten mit einer dentalen Lichtquelle Spectramat (Ivoclar Vivadent AG) bestrahlt und damit ausgehärtet wurden. Analog der ISO-Norm ISO-4049 (Dentistry - Polymer-based filling, restorative and luting materials) wurde die Biegefestigkeit bzw. der Biege-E-Modul bestimmt.

Aus Tabelle 2 ist ersichtlich, dass das Material B im Vergleich zum Material A zu vergleichbaren mechanischen Eigenschaften führt. Aufgrund der geringen Viskosität der McNano-1 Kieselsäurekondensate lässt sich ein höherer Füllgrad und damit eine weitere Verbesserung der Kompositeigenschaften erreichen.

## Patentansprüche

1. Dentalmaterialien enthaltend flüssige, funktionalisierte hydrophobe Cokondensate von Kieselsäuretetraalkylestern mit funktionalisierten Trialkoxysilanen der Formel I
**P-(CH2)ₙ-Si(OR)₃**
wobei P, R und n unabhängig voneinander die folgenden Bedeutungen haben:
P = eine polymerisationsfähige CH₂=CH-, CH₂=CH-O-, Styryl-, (Meth)acryl-, (Meth)acrylamid- oder eine polyadditionsfähige SH-Gruppe darstellt,
R = Methyl, Ethyl oder Propyl bedeutet und
n = von 3 bis 15 ist,
herstellbar durch Cokondensation von Kieselsäuretetraalkylestern mit in omega-Stellung funktionalisierten Alkyltrialkoxysilanen und anschließender Endgruppenfunktionalisierung mit Trimethylsilylgruppen.

2. Dentalmaterialien nach Anspruch 1,
**dadurch gekennzeichnet, dass** die Kondensate mit Trimethylsilylierungsmittel endgruppenfunktionalisiert sind.

3. Dentalmaterialien nach einem der vorhergehenden Ansprüche,
**dadurch gekennzeichnet, dass** unter basischen Bedingungen kondensiert wird.

4. Dentalmaterialien nach einem der vorhergehenden Ansprüche,
**dadurch gekennzeichnet, dass** als Trialkoxysilane Verbindungen der Formel I
**P-(CH₂)ₙ-Si(OR)₃**
verwendet werden, wobei P, R und n unabhängig voneinander die folgenden Bedeutungen haben:
P = eine polymerisationsfähige CH₂=CH-, (Meth)acryl-, (Meth)acrylamid- oder eine polyadditionsfähige SH-Gruppe darstellt,
R = Methyl oder Ethyl bedeutet und
n = von 3 bis 10 ist.

5. Dentalmaterialien nach einem der vorhergehenden Ansprüche erhältlich in einem 2-Schritt-Verfahren wobei
1. eine hydrolytische Kondensation von Kieselsäuretetraalkylester mit Silanen in Alkohol bei 30 bis 80°C unter Verwendung eines Katalysators durchgeführt wird und
2. die alkoholische Lösung des hydrolytischen Kondensates mit einer Lösung eines Trimethylsilylierungsmittels über einen Zeitraum von 10 bis 40 Stunden umgesetzt wird.

6. Dentalmaterialien nach einem der vorhergehenden Ansprüche,
**dadurch gekennzeichnet, dass** der Katalysator eine NH₄OH-Lösun darstellt.

7. Dentalmaterialien nach einem der vorhergehenden Ansprüche,
**dadurch gekennzeichnet, dass** Wasser in einer Menge von 0.5 bis 2.0 mol pro Alkoxysilangruppe zugesetzt wird.

8. Dentalmaterialien nach einem der vorhergehenden Ansprüche,
**dadurch gekennzeichnet, dass** das Trimethylsilylierungsmittel Trimethylchlorsilan ist.

9. Dentalmaterialien nach einem der vorhergehenden Ansprüche,
**dadurch gekennzeichnet, dass** sie Initiatoren zur Polymerisation der funktionalisierten hydrophoben Kieselsäurecokondensate enthalten, wobei sie als Initiatoren Photoinitiatoren und/oder Initiatoren für die thermische Härtung enthalten.

10. Dentalmaterialien nach einem der vorhergehenden Ansprüche,
**dadurch gekennzeichnet, dass** sie radikalisch polymerisierbare Monomere oder Polyadditionsharze enthalten.

11. Dentalmaterialien nach einem der vorhergehenden Ansprüche,
**dadurch gekennzeichnet, dass** sie organische oder anorganische Füllstoffpartikel enthalten.

12. Verwendung der Dentalmaterialien nach einem der vorhergehenden Ansprüche für Füllungskomposite, Befestigungszemente oder Materialien für Beschichtungen.

13. Verwendung der Dentalmaterialien nach einem der vorhergehenden Ansprüche für Füllüngskomposite enthaltend
a) 1 bis 45 Gew.-%, und besonders bevorzugt 1,00 bis 30 Gew.-% an in Hexan löslichen, nanopartikulären, funktionalisierten, hydrophoben Kieselsäurecokondensaten,
b) 0,01 bis 5 Gew.-%, besonders bevorzugt 0,1 bis 2,0 Gew.-% an radikalischem Initiator,
c) 5 bis 50 Gew.-% und besonders bevorzugt 0 bis 40 Gew.-% an anderen radikalisch polymerisierbaren Monomerkomponenten,
d) 30 bis 85 Gew.-% und besonders bevorzugt 40 bis 80 Gew.-% an einem Füllstoff.

14. Verwendung der Dentalmaterialien nach einem der vorhergehenden Ansprüche für Zemente enthaltend
a) 1 bis 60 Gew.-%, und besonders bevorzugt 1,0 bis 30 Gew.-% an in Hexan löslichen, nanopartikulären, funktionalisierten, hydrophoben Kieselsäurecokondensaten,
b) 0,01 bis 5 Gew.-%, besonders bevorzugt 0,1 bis 2,0 Gew.-% an radikalischem Initiator,
c) 5 bis 60 Gew.-% und besonders bevorzugt 0 bis 40 Gew.-% an anderen radikalisch polymerisierbaren Monomerkomponenten,
d) 20 bis 60 Gew.-% und besonders bevorzugt 30 bis 60 Gew.-% an einem Füllstoff.

15. Verwendung der Dentalmaterialien nach einem der vorhergehenden Ansprüche für Beschichtungsmaterialien enthaltend
a) 1 bis 95 Gew.-%, und besonders bevorzugt 10 bis 40 Gew.-% an in Hexan löslichen, nanopartikulären, funktionalisierten, hydrophoben Kieselsäurecokondensate,
b) 0,01 bis 5 Gew.-%, besonders bevorzugt 0,1 bis 2,0 Gew.-% an radikalischem Initiator,
c) 5 bis 60 Gew.-% und besonders bevorzugt 0 bis 40 Gew.-% an anderen radikalisch polymerisierbaren Monomerkomponenten,
d) 0 bis 20 Gew.-% an einem Füllstoff.

## Claims

1. Dental materials comprising liquid, functionalized hydrophobic co-condensates of tetraalkyl silicates with functionalized trialkoxysilanes of the formula I
**P-(CH₂)ₙ-Si(OR)₃**
where P, R and n independently of one another have the following meanings:
P = a polymerizable CH₂=CH-, CH₂=CH-O-, styryl, (meth)acrylic or (meth)acrylamide group or an SH group capable of polyaddition,
R = methyl, ethyl or propyl and
n = from 3 to 15,
which can be prepared by co-condensation of tetraalkyl silicates with alkyltrialkoxysilanes functionalized in the omega position and subsequent end group functionalization with trimethylsilyl groups.

2. Dental materials according to Claim 1, **characterized in that** the condensates are end group-functionalized with trimethylsilylating agent.

3. Dental materials according to one of the preceding claims,
**characterized in that** the condensation is carried out under basic conditions.

4. Dental materials according to one of the preceding claims,
**characterized in that** the trialkoxysilanes used are compounds of the formula I
**P-(CH₂)ₙ-Si(OR)₃**
where P, R and n independently of one another have the following meanings:
P = a polymerizable CH₂=CH-, (meth)acrylic or (meth)acrylamide group or an SH group capable of polyaddition,
R = methyl or ethyl and
n = from 3 to 10.

5. Dental materials according to one of the preceding claims obtainable in a 2-step process where
1. a hydrolytic condensation of tetraalkyl silicates with silanes is carried out in alcohol at 30 to 80°C using a catalyst and
2. the alcoholic solution of the hydrolytic condensate is reacted with a solution of a trimethylsilylating agent over a period of from 10 to 40 hours.

6. Dental materials according to one of the preceding claims,
**characterized in that** the catalyst is an NH₄OH solution.

7. Dental materials according to one of the preceding claims,
**characterized in that** water is added in an amount of from 0.5 to 2.0 mol per alkoxysilane group.

8. Dental materials according to one of the preceding claims,
**characterized in that** the trimethylsilylating agent is trimethylchlorosilane.

9. Dental materials according to one of the preceding claims,
**characterized in that** they contain initiators for the polymerization of the functionalized hydrophobic silicic acid co-condensates, wherein they contain photoinitiators and/or initiators for thermal curing as initiators.

10. Dental materials according to one of the preceding claims,
**characterized in that** they contain monomers polymerizable by free radicals or polyaddition resins.

11. Dental materials according to one of the preceding claims,
**characterized in that** they contain organic or inorganic filler particles.

12. Use of the dental materials according to one of the preceding claims for filling composites, fixing cements or materials for coatings.

13. Use of the dental materials according to one of the preceding claims for filling composites comprising
a) 1 to 45% by weight, and particularly preferably 1.00 to 30% by weight, of hexane-soluble, nanoparticulate, functionalized, hydrophobic silicic acid co-condensates,
b) 0.01 to 5% by weight, particularly preferably 0.1 to 2.0% by weight, of free radical initiator,
c) 5 to 50% by weight and particularly preferably 0 to 40% by weight of other monomer components polymerizable by free radicals,
d) 30 to 85% by weight and particularly preferably 40 to 80% by weight of a filler.

14. Use of the dental materials according to one of the preceding claims for cements comprising
a) 1 to 60% by weight, and particularly preferably 1.0 to 30% by weight, of hexane-soluble, nanoparticulate, functionalized, hydrophobic silicic acid co-condensates,
b) 0.01 to 5% by weight, particularly preferably 0.1 to 2.0% by weight, of free radical initiator,
c) 5 to 60% by weight and particularly preferably 0 to 40% by weight of other monomer components polymerizable by free radicals,
d) 20 to 60% by weight and particularly preferably 30 to 60% by weight of a filler.

15. Use of the dental materials according to one of the preceding claims for coating materials comprising
a) 1 to 95% by weight, and particularly preferably 10 to 40% by weight, of hexane-soluble, nanoparticulate, functionalized, hydrophobic silicic acid co-condensates,
b) 0.01 to 5% by weight, particularly preferably 0.1 to 2.0% by weight, of free radical initiator,
c) 5 to 60% by weight and particularly preferably 0 to 40% by weight of other monomer components polymerizable by free radicals,
d) 0 to 20% by weight of a filler.

## Revendications

1. Matériaux dentaires contenant des produits liquides hydrophobes, fonctionnalisés, de co-condensation de silicates de tétraalkyle avec des trialcoxysilanes fonctionnalisés de formule I
**P-(CH₂)ₙ-Si(OR)₃**
dans laquelle P, R et n ont, chacun indépendamment, les significations suivantes:
P représente un groupe CH₂=CH, CH₂=CH-O, styryle, (méth)acryloyle, (méth)acrylamido polymérisable ou un groupe SH apte à la polyaddition,
R représente le groupe méthyle, éthyle ou propyle et
n vaut de 3 à 15,
pouvant être préparés par co-condensation de silicates de tétraalkyle avec des alkyltrialcoxysilanes fonctionnalisés en position oméga et fonctionnalisation subséquente des groupes terminaux avec des groupes triméthylsilyle.

2. Matériaux dentaires selon la revendication 1, **caractérisés en ce que** les produits de condensation sont fonctionnalisés sur les groupes terminaux avec des agents de triméthylsilylation.

3. Matériaux dentaires selon l'une quelconque des revendications précédentes, **caractérisés en ce que** la condensation est effectuée dans des conditions basiques.

4. Matériaux dentaires selon l'une quelconque des revendications précédentes, **caractérisés en ce qu'**on utilise comme trialcoxysilanes des composés de formule I
**P-(CH₂)ₙ-Si(OR)₃**
dans laquelle P, R et n ont, chacun indépendamment, les significations suivantes:
P représente un groupe CH₂=CH, (méth)acryloyle, (méth)acrylamido polymérisable ou un groupe SH apte à la polyaddition,
R représente le groupe méthyle ou éthyle et
n vaut de 3 à 10.

5. Matériaux dentaires selon l'une quelconque des revendications précédentes, pouvant être obtenus dans un procédé en 2 étapes, dans lequel
1. on effectue une condensation hydrolytique de silicate de tétraalkyle avec des silanes dans de l'alcool à une température de 30 à 80 °C avec utilisation d'un catalyseur et
2. on fait réagir la solution alcoolique du produit de condensation hydrolytique avec une solution d'un agent de triméthylsilylation pendant une durée de 10 à 40 heures.

6. Matériaux dentaires selon l'une quelconque des revendications précédentes, **caractérisés en ce que** le catalyseur représente une solution de NH₄OH.

7. Matériaux dentaires selon l'une quelconque des revendications précédentes, **caractérisés en ce que** l'eau est ajoutée en une quantité de 0,5 à 2,0 moles par groupe alcoxysilane.

8. Matériaux dentaires selon l'une quelconque des revendications précédentes, **caractérisés en ce que** l'agent de triméthylsilylation est le triméthylchlorosilane.

9. Matériaux dentaires selon l'une quelconque des revendications précédentes, **caractérisés en ce qu'**ils contiennent des amorceurs pour la polymérisation des produits de co-condensation d'acide silicique hydrophobes fonctionnalisés, les amorceurs qu'ils contiennent étant des photoamorceurs et/ou des amorceurs pour le durcissement thermique.

10. Matériaux dentaires selon l'une quelconque des revendications précédentes, **caractérisés en ce qu'**ils contiennent des monomères polymérisables par voie radicalaire ou des résines de polyaddition.

11. Matériaux dentaires selon l'une quelconque des revendications précédentes, **caractérisés en ce qu'**ils contiennent des particules de charges organiques ou inorganiques.

12. Utilisation des matériaux dentaires selon l'une quelconque des revendications précédentes pour des composites d'obturation, des ciments de fixation ou des matériaux pour revêtements.

13. Utilisation des matériaux dentaires selon l'une quelconque des revendications précédentes pour des composites d'obturation, contenant
a) 1 à 45 % en poids, et de façon particulièrement préférée 1,00 à 30 % en poids de produits de co-condensation d'acide silicique hydrophobes, fonctionnalisés, nanoparticulaires, solubles dans l'hexane,
b) 0,01 à 5 % en poids, de façon particulièrement préférée 0,1 à 2,0 % en poids d'amorceur radicalaire,
c) 5 à 50 % en poids et de façon particulièrement préférée 0 à 40 % en poids d'autres composants monomères polymérisables par voie radicalaire,
d) 30 à 85 % en poids et de façon particulièrement préférée 40 à 80 % en poids d'une charge.

14. Utilisation des matériaux dentaires selon l'une quelconque des revendications précédentes pour des ciments, contenant
a) 1 à 60 % en poids, et de façon particulièrement préférée 1,0 à 30 % en poids de produits de co-condensation d'acide silicique hydrophobes, fonctionnalisés, nanoparticulaires, solubles dans l'hexane,
b) 0,01 à 5 % en poids, de façon particulièrement préférée 0,1 à 2,0 % en poids d'amorceur radicalaire,
c) 5 à 60 % en poids et de façon particulièrement préférée 0 à 40 % en poids d'autres composants monomères polymérisables par voie radicalaire,
d) 20 à 60 % en poids et de façon particulièrement préférée 30 à 60 % en poids d'une charge.

15. Utilisation des matériaux dentaires selon l'une quelconque des revendications précédentes pour des matériaux de revêtement, contenant
a) 1 à 95 % en poids, et de façon particulièrement préférée 10 à 40 % en poids de produits de co-condensation d'acide silicique hydrophobes, fonctionnalisés, nanoparticulaires, solubles dans l'hexane,
b) 0,01 à 5 % en poids, de façon particulièrement préférée 0,1 à 2,0 % en poids d'amorceur radicalaire,
c) 5 à 60 % en poids et de façon particulièrement préférée 0 à 40 % en poids d'autres composants monomères polymérisables par voie radicalaire,
d) 0 à 20 % en poids d'une charge.
